# EUROPÄISCHE PATENTANMELDUNG

(11) **EP 2 653 169 A1**
(43) Veröffentlichungstag der Anmeldung: **23.10.2013**
(21) Anmeldenummer: 13163280.4
(22) Anmeldetag: 11.04.2013
(51) Int. Cl.: A61K 49/00

(54) **Färbemittel zur Hornhautfärbung**

(30) Priorität: 11.04.2012 DE 102012103097; 09.11.2012 DE 102012110745
(71) Anmelder: FLUORON GMBH, 89077 Ulm (DE)
(72) Erfinder: Kugler, Wilfried, 89077 Ulm (DE); Starnecker, Gerhard, 89077 Ulm (DE)
(74) Vertreter: Schiweck, Weinzierl & Koch

(57) **Zusammenfassung**

Die vorliegende Erfindung betrifft ein Färbemittel zur Einfärbung einer ophthalmischen Membran, insbesondere der Hornhaut eines menschlichen oder tierischen Auges. Insbesondere betrifft die Erfindung die Verwendung einer wässrigen, physiologisch kompatiblen Lösung, in welcher ein Farbstoff gelöst ist, zum Anfärben von Membranen, insbesondere der Hornhaut, oder Teilen davon, im menschlichen oder tierischen Auge.

## Beschreibung

### Gebiet der Erfindung

Die vorliegende Erfindung betrifft ein Färbemittel zur Einfärbung einer ophthalmischen Membran, insbesondere der Hornhaut eines menschlichen oder tierischen Auges. Insbesondere betrifft die Erfindung die Verwendung einer wässrigen, physiologisch kompatiblen Lösung, in welcher ein Farbstoff gelöst ist, zum Anfärben von Membranen, insbesondere der Hornhaut, oder Teilen davon, im menschlichen oder tierischen Auge.

### Hintergrund der Erfindung

Die Verwendung von Vitalfarbstoffen, um die Viabilität von Endothelzellen in Spenderhornhäuten, z.B. aus Spenderbanken, zu überprüfen, ist seit langem bekannt (A comparison of two different staining methods for evaluating corneal endothelial viability. Stocker F.W. et al., Arch Ophthalmol. 1966; 76(6): 833-835). Der Vorteil der Vitalfärbung gegenüber anderen Verfahren ist die schnelle und einfache Bestimmung von abgestorbenen Endothelzellen in der Hornhaut.

Für die Vitalfärbung von Spenderhornhäuten wird in vielen Hornhautbanken Trypanblau verwendet, diese aus der Klasse der Diazo-Farbstoffe bekannte Verbindung färbt den Kern von beschädigten oder toten Endothelzellen in den Spenderhornhäuten an (Evaluation of the endothelium of human donor corneas by induced dilation of intercellular spaces and trypan blue. Sperling S., Graefes Arch Clin Exp Ophthalmol. 1986; 224(5): 428-434). Die Verlässlichkeit der Trypanblau-Färbung bei Endothelzellzählungen wurde jedoch angezweifelt (Value of two mortality assessment techniques for organ cultured corneal endothelium: trypan blue versus TUNEL technique. Gain P. et al., Br J Ophthalmol. 2002; 86(3): 306-310).

Bei Trypanblau handelt es sich um eine zytotoxische Substanz, wie beispielsweise aus Veckeneer M. et al.: Ocular toxicity study of trypan blue injected into the vitreous cavity of rabbit eyes, Graefe's Arch Clin Ex Ophthalmol (2002) 239: 698-704 und Rezai K. A. et al.: Trypan Blue Induces Apoptosis in Human Retinal Pigment Epithelial Cells, Am J Ophthalmol (2004) 138: 492-495 bekannt ist.

Eine weitere schnelle und einfache Vitalfärbung für die Anfärbung von Spenderhornhäuten bietet der Test mit Janusgrün, der die Zellkerne und Mitochondrien geschädigter Zellen anfärbt (A new test for endothelial viability. The Janus green photometry technique. Hartmann C. et al.; Arch Ophthalmol. 1989 ;107(10): 1511-1515).

Die Vitalfärbung mit Indocyaningrün zur Bestimmung der Viabilität von Endothelzellen der Spenderhornhäute ist ebenfalls lange bekannt (Indocyanine green: a new vital stain for use before penetrating keratoplasty. McEnerney J.K. et al., Arch Ophthalmol. 1978; 96(8): 1445-1447), konnte sich aber nicht gegen die Verwendung von Trypanblau durchsetzen.

Der Farbstoff Alizarin Rot wurde in Laborstudien als Zusatz zu Trypanblau in Zweifachfärbungen getestet und als nützlich zur Unterscheidung von lebenden und toten Zellgrenzen postuliert (Dual staining of corneal endothelium with trypan blue and alizarin red S: importance of pH for the dye-lake reaction. Taylor M.J. et al., Br J Ophthalmol. 1981;65(12):815-819).

Ein weiterer in der Vitalitätsprüfung von Spenderhornhäuten verwendeter Farbstoff ist Fluorescein Diacetat, dieser färbt ebenfalls tote Hornhautendothelzellen an (Differential value of various vital stains of corneal endothelium. Wilhelm F. et al., Ophthalmologe. 1995; 92(4): 496-498). Auch dieser Farbstoff konnte sich allerdings nicht gegen die Verwendung von Trypanblau durchsetzen.

Bengalrosa galt jahrzehntelang als ein Farbstoff, der abgestorbene Epithelzellen der Hornhaut anzufärben vermag. Neuere Untersuchungen haben aber gezeigt, dass Bengalrosa auch in intakte Zellen einzudringen vermag und diese dann anfärbt (Patient tolerance and ocular surface staining characteristics of lissamine green versus rose bengal. Manning et al., Ophthalmology 1995; 102: 1953-1957). Bengalrosa hat sich in der Vergangenheit als zytotoxisch herausgestellt. Die Zytotoxizität wird durch Wechselwirkung mit Licht zusätzlich gesteigert (Evaluation of the Effect of Lissamine Green and Rose Bengal on Human Corneal Epithelial Cells, Kim, J. et al.; Cornea 1999; 18: 328-332).

Gefärbte Strukturen erlauben neben der obenstehenden Anwendung bei der Bestimmung der Vitalität von Spenderhornhäuten auch eine bessere Visualisierung während eines operativen Eingriffs und tragen dazu bei, das Operationsrisiko für den Patienten deutlich zu senken und das postoperative Ergebnis deutlich zu steigern. Neue Techniken in der Hornhauttransplantationschirurgie, wie z.B. die Anteriore lamellierende Keratoplastik (DALK = Deep anterior lamellar keratoplasty), oder die Posteriore lamellierende Keratoplastik (DLEK = Deep lamellar endothelial keratoplasty) wie z.B. DMEK (Descemet's membrane endothelial keratoplasty) und DSAEK (Descemet's stripping with automated endothelial keratoplasty) führen zu besseren Operationsergebnissen. Diese lamellierenden Techniken setzen jedoch eine genaue Bestimmung der einzelnen Hornhautschichten voraus. Vitalfarbstoffe können die Identifikation dieser Schichten durch deren spezielle Anfärbung unterstützen bzw. ermöglichen, indem sie spezifische Strukturen, wie z.B. die Collagenfasern des Stromas, die Endothelzellen oder die Descemet-Membran in der Spender- sowie der Empfängerhornhaut färben können.

In der Keratoplastik wurde bisher Trypanblau bei der perforierenden und der tiefen lamellierenden Keratoplastik eingesetzt (Use of trypan blue for penetrating keratoplasty. Roos J.C.; J Cataract Refract Surg. 2005; 31(10): 1867-1869; Deep lamellar keratoplasty with trypan blue intrastromal staining. Balestrazzi E.; J Cataract Refract Surg. 2002; 28(6): 929-931). Trypanblau wurde als sinnvolles intra-operatives Färbemittel postuliert, um posteriore stromale Schichten sichtbar zu machen.

Wie bereits hierin beschrieben, handelt es sich bei Trypanblau um eine zytotoxische Substanz. Bei der Verwendung von Trypanblau ist daher die vollständige Ausspülung insbesondere des Augenbereiches, in welchem das Trypanblau als Färbemittel zum Einsatz gekommen ist, unmittelbar nach der Operation erforderlich, um einen längeren Verbleib im Körper bzw. im Auge zu vermeiden.

Ein weiterer Farbstoff, welcher im DLEK Verfahren getestet wurde, ist Indocyaningrün (ICG). Hierbei wurde das zu transplantierende Hornhautstroma des Spenders gefärbt (Use of indocyanine green in deep lamellar endothelial keratoplasty. John T.; J Cataract Refract Surg. 2003; 29(3): 437-443).

Es ist jedoch bekannt, dass ICG fototoxisch (Phototoxicity of Indocyanine Green on Human Retinal Pigment Epithelium in Vitro and its Reduction by Lutein. Wu W.-C. et al., Photochemistry and Photobiology 2005; 81: 537-540) und zytotoxisch (Comparative studies on the retinal toxicity of trypan blue and indocyanin green. Ito T. et al., Invest Ophthalmol Vis Sci 2004; 45: 3669-B130) ist.

Gentianaviolett wurde als Färbemittel für die periphere stromale Oberfläche mit Descemet-Membran und für das Endothelium im DLEK-Verfahren vorgeschlagen (Simple technique to unfold the donor corneal lenticule during Descemet's stripping and automated endothelial keratoplasty. Koenig S.B., J Cataract Refract Surg. 2007; 33(2): 189-190). Gentanaviolett ist jedoch bekannt dafür Hornhautödeme zu verursachen (Gentian violet solution for staining the anterior capsule. Ünlü K. et al.; J Cataract Refract Surg. 2000; 26: 1228-1232 und Experimental staining of the anterior lens capsule in albino rabbits, Gamal Eldin et. al.; J Cataract Refract Surg. 1999; 25: 1289-1294).

Zur Anfärbung einer Inzision in eine klare Hornhaut wurde die Verwendung von einer mit Trypanblau beschichteten Klinge vorgeschlagen, um die Inzision besser visualisierbar zu machen (Clear corneal incision with trypan-bluecoated blades. Kayikcioglu O.; J Cataract Refract Surg. 2007; 33(2): 351-352).

Farbstoffe, welche konventionell verwendet werden, um die Vitalfärbung von Spenderhornhäuten durchzuführen, oder postuliert wurden, um in operativen Verfahren an der Hornhaut, wie z.B. Verfahren der Hornhauttransplantation, eingesetzt zu werden, weisen jedoch weiterhin Nachteile in Zytotoxizität, Färbeverhalten, Herstellung und/oder Verwendung auf. Berichte über potentielle Toxizität, Teratogenität usw., technische Probleme wie die Löslichkeit, und Probleme bei der Verwendung, wie komplizierte Färbetechniken, Pigmentierungsverhalten und Ausspülverhalten machen deren Verwendung in der Hornhautchirurgie nach wie vor zu einem schwierigen Gebiet.

Die bisherigen verwendeten Farbstoffe weisen wie bereits dargelegt teilweise ein ungünstiges Sicherheitsprofil auf, so dass bis heute keiner dieser Farbstoffe für die Verwendung in der Hornhautchirurgie zugelassen wurde. Hinzu kommt teilweise eine gewisse Unspezifität der Farbstoffe. Um nicht von einem einzigen Farbstoff abhängig zu sein, ist es sinnvoll, parallel nach weiteren Alternativen, evtl. besser verträglichen Farbstoffen, zu suchen.

Aufgrund dessen besteht der Wunsch nach der Entwicklung eines Färbemittels für die ophthalmische Chirurgie, insbesondere für die Hornhautfärbung bzw. eines Färbemittels zur Verbesserung der ophthalmischen Chirurgie, insbesondere bei der Hornhautfärbung, wobei dieses Färbemittel wünschenswerterweise gute Färbeeigenschaften gepaart mit hoher Biokompatibilität/fehlender Zytotoxizität aufweisen soll.

### Beschreibung der Erfindung

Aufgabe der Erfindung ist daher die Bereitstellung eines Färbemittels, welches den vorbeschriebenen Vorstellungen bzw. Bedürfnissen im Stand der Technik nachkommt. Dieses Färbemittel soll zur Sichtbarmachung von ophthalmischen Membranen, insbesondere der Hornhaut oder Teilen davon, bzw. deren Bestandteile, geeignet sein.

Diese Aufgabe wird erfindungsgemäß durch die in der vorliegenden Anmeldung beschriebene Erfindung, insbesondere durch die Merkmale der unabhängigen Ansprüche gelöst. Die Unteransprüche und die vorliegende Anmeldung beinhalten weiterhin bevorzugte Ausführungsformen der Erfindung.

Es wird, wie in den Ansprüchen definiert, ein biokompatibles Färbemittel zum Färben der Hornhaut oder Teilen davon, bzw. deren Bestandteile zur Verfügung gestellt, welches als Hauptkomponente mindestens einen Farbstoff enthält, der ausgewählt ist aus Triphenylmethanfarbstoffen und/oder Azofarbstoffen, Cyaninfarbstoffen, Naphtocyaninfarbstoffen, wie in der WO 2011/151287 beschrieben, und/oder Naturfarbstoffen, oder ein pharmazeutisch verträgliches Salz und/oder Hydrat dieser Farbstoffe, sowie einen pharmazeutisch akzeptablen Träger. Das erfindungsgemäße Färbemittel wird durch Lösen des Farbstoffs in einer wässrigen Lösung hergestellt. Die wässrige Lösung enthält vorzugsweise einen Puffer, z.B. einen Hydrogenphosphatpuffer, bestehend aus Dinatriumhydrogenphosphat und Natriumdihydrogenphosphat, Natriumchlorid und Wasser.

Überraschenderweise wurde gefunden, dass die Verwendung von Acid Violet 17 in einem Färbemittel zur Färbung von ophthalmischen Membranen nicht nur eine Alternative, sondern eine vielschichtige Verbesserung gegenüber dem Stand der Technik zeigt. So wurde insbesondere gefunden, dass das erfindungsgemäße Färbemittel sowohl aus dem Auge zu entfernende ophthalmische Membranen, als auch die vordere und hintere Linsenkapsel färbt und zudem in der Lage ist, auch die Hornhaut, Teile davon bzw. deren Bestandteile zu färben. Diese gebündelte Färbekapazität konnte nicht erwartet werden, da sie bislang noch bei keinem bekannten Färbemittel beobachtet worden war. Gleiches trifft für Brillant Blau g (BBG) zu. Daher sind die beiden Farbstoffe Acid Violet 17 und BBG, insbesondere BBG, aber auch im Allgemeinen biokompatible Triphenylmethanfarbstoffe im Sinne der Erfindung bevorzugte Farbstoffe.

Zudem wurde überraschend gefunden, dass Acid Violet 17 sehr gut in solchen Flüssigkeiten löslich ist, die sowohl zur Anwendung im vorderen als auch hinteren Bereich des Auges geeignet sind, wie z.B. Balanced Salt Solution (BBS), Wasser zur Injektion, etc. Überdies ist Acid Violet 17 auch in solchen Flüssigkeiten sehr gut löslich, die schwerer als Wasser, also ≥ 1,01 g/cm³ sind, wie z.B. Polyethylenglykol. Die gute Löslichkeit von Acid Violet 17 bringt mit sich, dass sich eine erfindungsgemäße Farbstofflösung gut herstellen lässt, weil keine Klümpchen oder Ausflockungen, also unlösliche Farbstoffkristalle/-partikel auftreten. Gleiches gilt für BBG.

Dementsprechend gut handhabbar ist auch die Anwendung des erfindungsgemäßen Färbemittels bei einem hierin beschriebenen Verfahren der Augenchirurgie. Dort ist es außerordentlich wichtig, dass es bei der Applikation, insbesondere beim Applizieren des Farbstoffs, nicht zur Klümpchen-Bildung oder Ausflockungen kommt, da ansonsten die Kanüle der Spritze mit dem Färbemittel verstopfen würde, wodurch der Operateur höheren Druck ausüben würde, so dass das Färbemittel im schlechtesten Fall mit zu hohem Druck verabreicht werden müsste, der letztendlich dazu führt, dass Zellen, Gewebe und/oder Strukturen im Auge geschädigt, ja sogar zerstört werden würden. Durch unlösliche Farbstoffkristalle/partikel welche während der Operation in das Patientenauge gelangen und eventuell dort verbleiben, könnten darüber hinaus auch mechanische und/oder immunologische Probleme entstehen.

Die bevorzugte gute Löslichkeit des erfindungsgemäßen Färbemittels führt auch dazu, dass das Färbemittel wieder gut auswaschbar ist. Eine gute Auswaschbarkeit ist wichtig, da überschüssiges Färbemittel aus dem Auge wieder entfernt werden sollte, um etwaige Spätschäden oder andere Komplikationen weitestgehend auszuschließen bzw. zu verhindern.

Die Färbequalitäten von BBG und Acid Violet 17 sind im Vergleich zu den gegenwärtig verwendeten Farbstoffen, wie Trypanblau (ERM-Färbung, Kapselfärbung) bzw. Blueron (Kapselfärbung), Indocyanin-Grün (ILM-Färbung), verbessert, somit kann BBG bzw. Acid Violet 17, also ein Triphenylmethanfarbstoff, wie hierin beschrieben, Objekte bei sehr niedrigen Farbstoffkonzentrationen färben. Die Verbesserung gegenüber dem Stand der Technik wird bedingt durch eine verbesserte Visualisierung, einer schnelleren Anfärbung der ophthalmischen Membranen, sowie einer kontrastreicheren Färbung bei niedriger Konzentration.

Es wurde zudem beobachtet, dass Acid Violet 17 vorzugsweise in der Lage ist sowohl die innere Grenzmembran (ILM) als auch die vordere und hintere Linsenkapsel anzufärben. Gleiches gilt für BBG. Bisher ist kein Farbstoff bekannt, welcher bei beiden ophthalmologischen Membranen eine entsprechende, in der ophthalmischen Chirurgie verwendbare Anfärbung zeigt und somit eine evtl. gleichzeitige Anfärbung der Membranen zulässt.

Darüber hinaus kann das erfindungsgemäße Färbemittel zusätzlich zum Anfärben der Hornhaut bzw. der hierin beschriebenen Schichten der Hornhaut, z.B. bei Hornhauttransplantationen, verwendet werden. Das heißt, dass das erfindungsgemäße Färbemittel in einer Ausführungsform die innere begrenzende Membran (Membrana limitans interna) (ILM), die vordere und/oder hintere Linsenkapsel und/oder die Hornhaut bzw. deren Bestandteile anfärben kann.

Daher stellt die vorliegende Erfindung ein biokompatibles Färbemittel zur Verwendung in einem Verfahren zum Färben der Hornhaut bereit bzw. zur Verwendung in einem Verfahren der Hornhautchirurgie, z.B. Keratoplastik.

Ferner stellt die vorliegende Erfindung auch die Verwendung des biokompatiblen Färbemittels zum Färben der Hornhaut bereit, ebenso die Verwendung des biokompatiblen Färbemittels zur Herstellung eines Medizinprodukts oder diagnostischen Mittels zum Färben der Hornhaut bzw. zur Verwendung in einem Verfahren der Hornhautchirurgie, z.B. Keratoplastik.

Ein weiterer Aspekt der vorliegenden Erfindung ist ein biokompatibles Färbemittel zur Verwendung in einem augenheilkundlichen (ophthalmologischen) oder augenchirurgischen Verfahren (Verfahren der Augenchirurgie oder chirurgisches Verfahren der Augenheilkunde), insbesondere der Hornhautchirurgie. Vorzugsweise umfasst das Verfahren der Augenchirurgie das Anfärben der Hornhaut und Durchführen eines hierin beschriebenen Verfahrens der Augenchirurgie, insbesondere der Hornhautchirurgie, z.B. ein Verfahren der Keratoplastik, z.B. Hornhauttransplantationschirurgie, wie z.B. die anteriore lamellierende Keratoplastik (DALK = Deep anterior lamellar keratoplasty), oder die posteriore lamellierende Keratoplastik (DLEK = Deep lamellar endothelial keratoplasty) wie z.B. DMEK (Descemet's membrane endothelial keratoplasty) und DSAEK (Descemet's stripping with automated endothelial keratoplasty).

Ein anderer Aspekt der vorliegenden Erfindung ist ein Verfahren zum Anfärben der Hornhaut, umfassend Anfärben der Hornhaut und Durchführen eines hierin beschriebenen chirurgischen Verfahrens der Augenheilkunde. Ein bevorzugtes chirurgisches Verfahren ist die Hornhauttransplantation, umfassend Entfernen und/oder Einsetzen zumindest eines Teils einer mit dem erfindungsgemäßen Färbemittel gefärbten Hornhaut oder Teilen davon bzw. deren Bestandteile oder der gesamten Hornhaut. Dabei kann die Hornhaut oder Teile/Bereiche der Hornhaut des Spenders und/oder die Hornhaut oder Teile/Bereiche der Hornhaut des Empfängers mit dem erfindungsgemäßen Färbemittel angefärbt werden und so das chirurgische Verfahren an und/oder mit der angefärbten Hornhaut durchgeführt werden.

Ein noch weiterer Aspekt der vorliegenden Erfindung ist das Anfärben der Hornhaut mit dem erfindungsgemäßen Färbemittel, die sich in einer Hornhautbank befindet, um so die Orientierung der Hornhaut herauszufinden und/oder die Orientierung der Hornhaut zu verifizieren.

Ein wichtiger Bestandteil der erfindungsgemäßen Zubereitung ist der Farbstoff. Als Farbstoff können solche Verbindungen eingesetzt werden, die die Hornhaut bzw. Bestandteile davon spezifisch und gezielt anfärben können, so dass sich die Hornhaut bzw. deren Bestandteile optisch von anderen Strukturen unterscheiden. Weiterhin muss der Farbstoff in Wasser bzw. der Mischung aus Wasser und einem weiteren Lösungsmittel löslich sein. Er darf weder toxisch, insbesondere zytotoxisch bzw. zellschädigend sein, noch Schäden an der Hornhaut, z.B. Ödeme (wie Gentianaviolet, auch Enzianblau genannt) und/oder an der Netzhaut hervorrufen oder durch Lichtreaktionen toxische Wirkungen entwickeln wie ICG oder Trypanblau. Außerdem sollte er ein gutes Färbevermögen haben, um die Menge des Farbstoffs gering halten zu können.

Als vorteilhaft haben sich Farbstoffe aus der Gruppe der Triphenylmethanfarbstoffe, wie Acid Violet 17, Brilliant Blau G (BBG), Brilliant Blau R (BBR), Brilliant Blau FCF, Patentblau V, Bromphenolblau, Fast Green, Methylgrün, Brilliantsäuregrün, Rosanillin; aus der Gruppe der Azo- bzw. Diazofarbstoffe wie Orange G, Ponceau 2R, Chromotrope 6 R, Ponceau 6 R, Tartrazine, Azophloxine, Ponceau B1, Evans-Blau, Chicago Blau; aus der Gruppe der Cyaninfarbstoffe wie 3,3'-Diethylthiacyanin-iodid, 3,3'-Diethylthiacarbocyaniniodid, 3,3'-Diethyl-9-methylthiacarbocyanin-iodid, 1,1'-Diethyl-4,4'-cyanin-iodid und/oder aus der Gruppe der Naturfarbstoffe wie Orcein, Lawsone, Indigotin, Canthaxanthin, Hematoxylin, Indigocarmine, Anthocyanen, Anthocyanidine, Lutein, Zeaxanthin und/oder Anthraquinone, sowie deren Mischungen, d.h. Mischungen aus mehreren Mitgliedern einer der genannten Gruppen als auch aus Mitgliedern unterschiedlicher Gruppen, erwiesen.

Bevorzugt werden Triphenylmethanfarbstoffe verwendet. Von den Triphenylmethanfarbstoffen ist BBG oder Acid Violet 17 besonders bevorzugt.

Acid Violet 17 ist ein Triphenylmethanfarbstoff, der in der Textilindustrie als Farbstoff dient. Außerdem wird er in der Biochemie zum Anfärben von Proteinen bei der Polyacrylamid-Gelelektrophorese genutzt.

"Acid Violet 17" wird auch als "42650" oder "Coomassie Violet" bezeichnet, wobei Coomassie Violet Coomassie Violet R200 und R150 umfasst. Acid Violet 17 hat die Summenformel C41H45N3O6S2 und ein bevorzugtes Molekulargewicht von 739,94 g/mol. Die CAS-Nummer ist 4129-84-4. Die weiteren Bezeichnungen von Acid Violet 17 können alternativ zu dem Begriff Acid Violet 17 im Zusammenhang mit der vorliegenden Erfindung verwendet werden. Acid Violet 17 kann z.B. von Sigma Aldrich bezogen werden

Acid Violet 17 hat folgende Strukturformel:

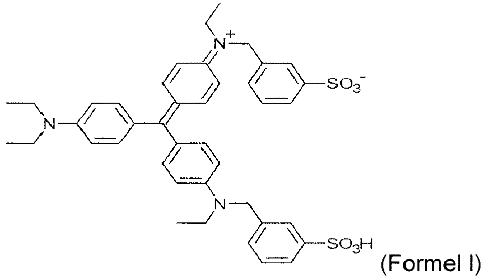

Das Natriumsalz von Formel I ist bevorzugt:

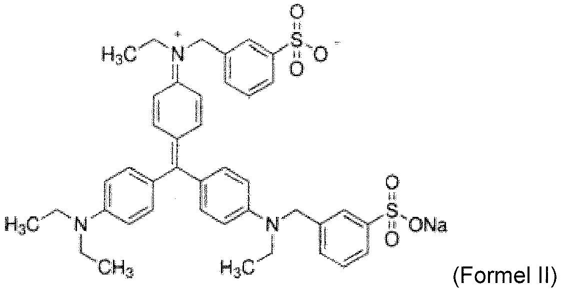

Jede dieser beiden Strukturformeln (Formel I bzw. II) kann auch alternativ zu dem Begriff "Acid Violet 17" im Zusammenhang mit der vorliegenden Erfindung verwendet werden.

Das erfindungsgemäß verwendete Acid Violet 17, bzw. sein pharmazeutisch verträgliches Salz und/oder Hydrat davon, kann durch in der Literatur bekannte Verfahren hergestellt werden.

Erfindungsgemäße Triphenylmethanfarbstoffe sind Abkömmlinge des farblosen Triphenylmethans. Von diesem lässt sich folgende Gruppierung, als gemeinsames Strukturmerkmal und Chromophor der Triphenylmethanfarbstoffe, ableiten:

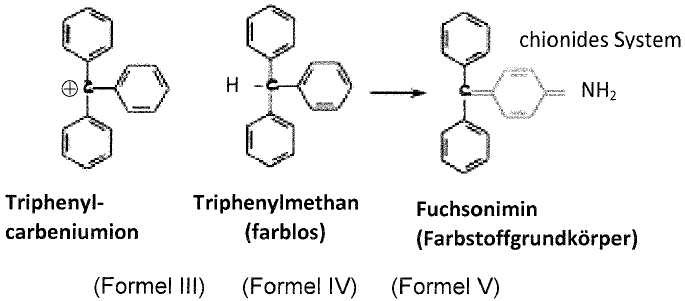

Mindestens zwei der drei Phenylsubstituenten sind bei Triphenylmethanfarbstoffen mit elektronenliefernden auxochromen bzw. antiauxochromen Gruppen substituiert. Das pi-Elektronensystem erstreckt sich dadurch über alle drei Benzolkerne und das zentrale sp2-hybridisierte Kohlenstoffatom unter Ausbildung eines chinoiden Systems. Dadurch entsteht dann ein ausgedehntes mesomeres pi-Elektronensystem, das sich über alle drei Ringe erstreckt und den eigentlichen Farbstoffgrundkörper, das Fuchsonimin bildet. Je nach den vorhandenen Auxochromen bzw. Antiauxochromen als Substituenten an den einzelnen Kernen sind einzelne Kerne stärker oder schwächer an der Mesomerie beteiligt, wobei dadurch das pi-Elektronensystem unterschiedlich beeinflusst und die Farbe der verschiedenen Verbindungen verändert wird. Jeder der drei Phenylsubstituenten des Triphenylmethangrundgerüsts kann wahlweise ein oder mehrere weitere Substituenten enthalten.

Bei auxochromen Gruppen handelt es sich um Substituenten mit freien Elektronenpaaren am chromophoren System, die mit einem +M-Effekt an der Mesomerie des Elektronensystems teilnehmen und somit eine weitere Ausdehnung der pi-Elektronenwolke bewirken. Durch die verstärkte Delokalisation können die Elektronen noch leichter angeregt werden. Unter dem Einfluss der Substituenten findet eine Verschiebung der Absorption zum längeren Wellenbereich hin statt und man erhält eine Farbvertiefung (Bathochromie). In der Reihenfolge abnehmender Farbverstärkung sind das u.a.: -NR2, -NHR, -NH2, -OR, - Halogen, und -OH. Das Einwirken von auxochromen Gruppen bewirkt neben der Farbvertiefung auch eine Bindung des Farbstoffs zum färbenden Material.

Eine weitere Delokalisierung der pi-Elektronen kann zusätzlich durch Substituenten am mesomeren System hervorgerufen werden, die als Elektronenakzeptoren wie z.B. C=O, - NO2 und N=N wirken und den pi-Elektronensystems Elektronen entziehen. Eine solche Gruppe bezeichnet man als antiauxochrom, da sie entgegen auxochromen Gruppen wirkt.

Entsprechend einer bevorzugten Ausführungsform der vorliegenden Erfindung, wird zum Anfärben der Hornhaut, bzw. deren Bestandteile bei der Hornhautchirurgie ein biokompatibles Färbemittel, enthaltend einen Farbstoff ausgewählt aus Triphenylmethanfarbstoffen, und/oder Azofarbstoffen und/oder Cyaninfarbstoffen und/oder Naturfarbstoffen wie Anthocyanen und Anthocyanidinen, und/oder ein pharmazeutisch verträgliches Salz und/oder Hydrat davon als Hauptkomponente bereitgestellt.

Beispiele für pharmazeutisch verträgliche Salze beinhalten Salze mit anorganischen Basen, Ammoniak, organischen Basen, anorganischen Säuren, organischen Säuren, basischen Aminosäuren, Halogenionen oder dergleichen, sowie innere molekulare Salze. Beispiele einer anorganischen Base umfassen Alkalimetall (z.B. Na, K) und Erdalkalimetall (z. B. Ca, Mg). Beispiele der organischen Base umfassen Trimethylamin, Triethylamin, Cholin, Procain, Ethanolamin und dergleichen. Beispiele der anorganischen Säure umfassen Salzsäure, Bromwasserstoffsäure, Schwefelsäure, Salpetersäure und Phosphorsäure und dergleichen. Beispiele der organischen Säure umfassen p-Toluolsulfonsäure, Methansulfonsäure, Ameisensäure, Trifluoressigsäure, Maleinsäure und dergleichen. Beispiele für die basische Aminosäure umfassen Lysin, Arginin, Ornithin, Histidin und dergleichen. Die Verbindung kann auch ein pharmazeutisch annehmbares Hydrat sein.

Entsprechend einer bevorzugten Ausführungsform der vorliegenden Erfindung kann das Färbemittel als chirurgisches Hilfsmittel bei augenheilkundlichen Operationen, betreffend ophthalmische Erkrankungen, wie zum Beispiel, Erkrankungen des Glaskörpers und der Netzhaut, wie Makula-Loch, Netzhautablösung bedingt durch ein hochmyopes Auge, epiretinale Membran, proliferative diabetische Retinopathie, makuläres Ödem wie z.B. diabetisches makuläres Ödem, und proliferative Vitreo-Retinopathie, sowie spezifische Katarakte, wie hypermaturer Katarakt und kongenitaler Katarakt, darüber hinaus kann das auch bei der perforierenden Keratoplastik, der vorderen und hinteren lamellierenden Keratoplastik und der Corneamarkierung, etc., benutzt werden. Aufgrund der Farbzusammensetzung der vorliegenden Erfindung, wird es möglich, die nur schwer erkennbaren ophthalmischen Membranen klarer sichtbarzumachen, und die Sicherheit während chirurgischer Eingriffe zu erhöhen.

In einer weiteren bevorzugten Ausführungsform der vorliegenden Erfindung kann das Färbemittel genutzt werden, um eine ophthalmische Membran zu färben, besonders bevorzugt um die innere Grenzmembran, die vordere und hintere Linsenkapsel und/oder die Hornhaut bzw. deren Bestandteile zu färben.

Die ophthalmische Membran kann auch die Hornhaut des Auges sein, aber sie ist nicht notwendigerweise auf diese begrenzt. Beim Färben der Hornhaut verhält es sich so, dass der ausführende Operateur die Hornhaut des Auges des Transplantatempfängers markieren kann, um dann entsprechende Stellen auszuschneiden, z.B. mechanisch oder mittels Excimer-Laser Trepanation und mit einem Transplantat zu ersetzen. Es können bei der Hornhauttransplantation alle Schichten der Hornhaut transplantiert werden (perforierende Keratoplastik) oder nur spezielle Schichten der Hornhaut (lamellierende Keratoplastik). Um nun die Hornhaut bzw. Bereiche oder Stellen sowohl der Spender-Hornhaut als auch der Akzeptor-Hornhaut davon sichtbar zu machen, kann das erfindungsgemäße Färbemittel verwendet werden um z.B. die Hornhaut von außen zu markieren oder beim Vernähen der Spenderhornhaut mit der Empfängerhornhaut als Nahtmarker zu dienen.

Entsprechend einer mehr bevorzugten Ausführungsform der vorliegenden Erfindung kann das Färbemittel als chirurgisches Hilfsmittel bei augenheilkundlichen Operationen, wie z.B. der Thermokeratoplastik, der perforierenden Keratoplastik, der vorderen und hinteren lamellierenden Keratoplastik und der Corneamarkierung, der Epikeratophakie, etc., betreffend ophthalmische Erkrankungen, wie zum Beispiel Erkrankungen der Hornhaut, wie Hornhautdystrophien (z.B. Reis-Bücklersche Hornhautdystrophie und Fuchs-Hornhautendotheldystrophie), bullöse Keratopathien (z.B. pseudophake bullöse Keratopathie und Keratopathie beim Pseudoexfoliationssyndrom), Hornhautentzündungen/Keratitis (z.B. metaherpetische Keratitis), oberflächliche Hornhautnarben nach Verletzungen, Infektionen oder Verätzungen der Hornhaut, drohende Perforation bei brüchigem oder ausgedünntem Hornhautgewebe, exophytisch wachsende Tumoren und Pterygien, Gewebedefekte nach Entfernung von Dermoiden der Hornhaut bei Kindern, benutzt werden. Aufgrund der Farbzusammensetzung der vorliegenden Erfindung, wird es möglich, die nur schwer erkennbaren Strukturen der Hornhaut klarer sichtbarzumachen, und die Sicherheit während chirurgischer Eingriffe zu erhöhen.

Eine "ophthalmische Membran" ist eine Membran im Auge bzw. eine solche Membran, die Strukturen im Auge begrenzt und/oder voneinander trennt und daher eine zelluläre Grenzschicht im Auge darstellt. Solche Grenzschichten gibt es in großer Zahl. Dazu gehören beispielsweise die Bruch-Membran, welche eine Grenzmembran zwischen der Choroidea (Aderhaut) und dem retinalen Pigmentepithel (RPE) des Auges ist; die Basalmembran, wobei alle Epithelgewebe auf einer Basalmembran ruhen, welche sie vom darunterliegenden Bindegewebe trennt; die Descemet-Membran, welche sich zwischen dem Hornhautstroma und dem Hornhautendothel befindet und auch unter der Bezeichnung Lamina limitans posterior bekannt ist; die Bowman-Membran, auch Bowman-Schicht oder Lamina limitans anterior, welche zwischen dem Hornhautstroma und der Basalmembran des Hornhautendothels liegt und ca. 12 µm dick ist; die Glaskörpergrenzmembran, wobei diese extrem feine Membran den gelartigen Glaskörper umgibt und vorne an die Linse und sonst an die Netzhaut grenzt und sich im Alter von diesen ablöst; die Epiretinale Membran, auch bekannt als Makular pucker oder als Zellophan-Retinopathie, sind pathologische (krankhafte) Membranentwicklungen, die charakterisiert sind durch die Entwicklung einer Membran über der Makula (zentrale Netzhaut); der Kapselsack der Linse, welche die Basalmembran der Linsenepithelzellen darstellt und die Augenlinse umschließt; die Bindehaut, welche die transparente Membran ist, die unser geöffnetes Auge abschließt; die Barkan Membran, welche ein persistierendes mesodermales Gewebe im Kammerwinkel bei kongenitalem Glaukom (angeborenem grünen Star) ist; die Pseudomembran, welche aus Zellen infolge einer chronischen Konjunktivitis besteht; die Table-Top Membran, welche auf der Makula sitzt; die Inner Limiting Membran (ILM, Membrana limitans interna), wobei sie als innere Grenzschicht der Retina die Basalmembran der Müllerschen Zellen der Netzhaut ist; die Outer Limiting Membran (ELM, Membrana limitans externa), welche die äußere Grenzschicht der Retina ist und sich zwischen dem Pigmentepithel und den Photorezeptoren (Stäbchen und Zapfen) der Netzhaut befindet; die Sekundärmembran, welche ein Nachstar oder auch postoperativer Katarakt ist, wobei es sich um eine fibrotische Veränderung der bei der Kataraktchirurgie im Auge belassenen, hinteren Linsenkapselmembran handelt; die entzündlichen Membranen, welche durch entzündliche Prozesse entstandene Eiweißschichten sind, die sich zahlreichen Strukturen im Auge auflagern können, z. B. Linse, Kunstlinse, hintere Linsenkapsel nach Staroperation, Ziliarkörper, etc.

Erfindungsgemäß bevorzugte ophthalmische Membranen sind die Retina (Netzhaut), die vordere und hintere Linsenkapsel, die innere begrenzende Membran (Membrana limitans interna) (ILM), die epiretinale Membran (ERM) oder die Hornhaut (Cornea). Die Hornhaut ist aus verschiedenen Schichten aufgebaut, insbesondere aus einem Epithel (=oberste Deckschicht), einer ersten Zwischenmembran (Bowmansche Membran oder Lamina limitans anterior), der Hauptsubstanz (=Stroma oder Hornhautparenchym), einer zweiten Zwischenmembran (=Descemetsche Membran oder Lamina limitans posterior) und der untersten Deckschicht (=Endothel). Diese Schichten sind auch von dem Begriff "ophthalmische Membran" umfasst.

"Aus dem Auge zu entfernende Membranen" oder "eine aus dem Auge zu entfernende Membran" bezeichnet eine ophthalmische Membran, vorzugsweise die innere begrenzende Membran (Membrana limitans interna) (ILM), die epiretinale Membran (ERM), die vordere und hintere Linsenkapsel und/oder die Hornhaut bzw. deren Bestandteile.

Zur Entfernung der epiretinalen Membran, wird vom erfindungsgemäßen Färbemittel das benachbarte Retinagewebe/ILM gefärbt. Beim Entfernen der Membran von dem darunter liegenden, nichteingefärbten Retinagewebe ergibt sich dann ein guter Kontrast. Nach dem Anfärben wird überschüssige Färbemittellösung ausgespült und der freie Raum durch BSS oder in der Ablatiochirurgie durch Luft, Gas oder Silikonöl angefüllt. Durch die Einfärbung ist es möglich, mit einem nicht beleuchteten oder nur schwach beleuchteten Instrument beim Abtragen (Peeling) der Membran zu arbeiten. Hierdurch wird bei ausreichender Kontrastwahrnehmung die Lichttoxizität erheblich verringert. Insbesondere bei der Anwendung im Zusammenhang mit epiretinaler Membranen (Epiretinale Gliose,"macula pucker", "surface wrinkling") bildet der Einsatz des erfindungsgemäßen Färbemittels eine wertvolle Hilfe beim Aufsuchen und Entfernen der Membranen.

Die Begriffe "Einfärben", "Anfärben", oder "Färben", wenn sie hierin verwendet werden, können gleichbedeutend verwendet werden. Jeder der Begriffe beinhaltet, dass eine ophthalmische Membran entweder selbst durch das erfindungsgemäße Färbemittel gefärbt/angefärbt wird (sog. Positivfärbung) und/oder dass zwar die ophthalmische Membran selbst nicht gefärbt wird, aber dafür ophthalmische Membranen, die um die nicht selbst gefärbte ophthalmische Membran gelegen sind und/oder an die selbst nicht gefärbte ophthalmische Membran angrenzen (sog. negative Färbung). Eine negative Färbung tritt zum Beispiel im Fall der ERM auf, wenn das erfindungsgemäße Färbemittel verwendet wird. Die Begriffe beinhalten auch, dass auch Strukturen außerhalb des Auges wie hierin beschrieben angefärbt werden.

Die Hornhaut (Cornea) ist aus verschiedenen Schichten aufgebaut, insbesondere aus einem Epithel (=oberste Deckschicht), einer ersten Zwischenmembran (Bowmansche Membran oder Lamina limitans anterior), der Hauptsubstanz (=Stroma oder Hornhautparenchym), einer zweiten Zwischenmembran (=Descemetsche Membran oder Lamina limitans posterior) und der untersten Deckschicht (=Endothel). Diese Schichten sind hierin als Teile oder Bestandteile der Hornhaut definiert. Mit dem Begriff "Hornhaut" ist die gesamte Hornhaut und/oder Teile davon bzw. deren Bestandteile gemeint.

In einer weiteren bevorzugten Ausführungsform der vorliegenden Erfindung kann das Färbemittel genutzt werden, um einen oder mehrere Bestandteile der Hornhaut zu färben, besonders bevorzugt ist hierbei die Anfärbung des Stromas z.B. die periphere stromale Oberfläche, die Descemet-Membran und das Endothelium.

Ein Färbemittel, ein pharmazeutisch verträgliches Salz und/oder Hydrat davon als Hauptkomponente kann ebenfalls außerhalb des Auges zum Färben von Sklera, Bindehaut, Sehnen, Muskeln und fibrotischem Gewebe, z.B. bei der Behandlung des Schielens (Strabismus) oder der posterioren Tenektomie, sowie zum Färben der Tenon-Kapsel z.B. bei der Enukleation genutzt werden.

Ein Färbemittel, ein pharmazeutisch verträgliches Salz und/oder Hydrat davon kann darüber hinaus zum Anfärben von Arzneimitteln wie z.B. antiproliferative Mittel bei Operationen am Auge, wie z.B. der Trabekulektomie im Bereich der Vorderkammer verwendet werden.

Überdies stellt die vorliegende Erfindung die Verwendung der hierin beschriebenen Farbstoffe zur Herstellung eines Färbemittels für die Visualisierung bzw. Anfärbung ophthalmischer Membranen bereit.

Außerdem wird gemäß eines weiteren Hauptaspekts der vorliegenden Erfindung die Verwendung der Farbstoffe als chirurgisches Hilfsmittel für augenheilkundliche Operationen zur Verfügung gestellt.

In einer bevorzugten Ausführungsform der vorliegenden Erfindung ist es bevorzugt, dass das Färbemittel den Farbstoff in einer Konzentration von 1 x 10⁻³ - 10 g/L beinhaltet, besonders bevorzugt ist eine Konzentration von 0,1 bis 1,5 g/L für die Färbung der Hornhaut bzw. deren Bestandteile. In dieser Ausführungsform wird ein Färbemittel mit einer hohen Färbeaffinität bei niedrigen Konzentrationen und geringen Mengen bereitgestellt und es wird eine spontane Einfärbung der gewünschten Bereiche im menschlichen oder tierischen Auge erreicht.

Entsprechend einer Ausführungsform der vorliegenden Erfindung ist bevorzugt, dass das Färbemittel in einer physiologisch kompatiblen wässrigen Lösung von insbesondere Natriumchlorid, die mit einem Puffer auf einen pH von 6,8 bis 7,8, insbesondere etwa 7,4 eingestellt sein kann, vorliegt. Als Puffer kann ein Phosphat-, Carbonat- oder Citrat-Puffer, dessen pH-Wert mittels Natriumhydroxid eingestellt werden kann, verwendet werden. Die Lösung kann bevorzugt eine intraokulare Spüllösung, eine ausgeglichene Salzlösung bzw. eine physiologische Kochsalzlösung sein.

Die erfindungsgemäße Zubereitung basiert in einer Ausführungsform auf Wasser als Lösungsmittel, wobei weitere Lösungsmittel gegebenenfalls in geringeren Anteilen enthalten sein können, sofern sie mit Wasser homogen vermischbar sind und biologisch kompatibel sind. In Betracht kommen hier ein- und mehrwertige Alkohole, wie sie im medizinischen Bereich auch Anwendung finden. Falls ein weiteres Lösungsmittel verwendet wird, ist dieses besonders bevorzugt ein Glycol oder Glycerin. Auch Mischungen der genannten Lösungsmittel kommen in Betracht. Falls dem Wasser ein Lösungsmittel zugemischt wird, so sollte dieses in einem Anteil von nicht mehr als 20 Gew.-%, bevorzugter nicht mehr als 10 Gew.-% verwendet werden.

Außer Wasser als Lösungsmittel und dem Farbstoff, enthält das erfindungsgemäße Färbemittel gegebenenfalls ein die Dichte einstellendes Mittel. Das die Dichte einstellende Mittel muss biologisch kompatibel sein, darf nicht toxisch sein und muss mit Wasser homogen vermischbar sein, ggf. nach Zugabe einer geringen Menge eines Löslichkeitsvermittlers wie Alkohol, so dass eine klare transparente Lösung entsteht. Außerdem muss es mit dem Farbstoff kompatibel sein, d.h. darf nicht die Löslichkeit des Farbstoffs in erheblichem Ausmaß beeinträchtigen.

In einer weiteren bevorzugten Ausführungsform der vorliegenden Erfindung wird die Dichte des Färbemittels daher in einem Bereich von 1,01 g/cm³ bis 1,50 g/cm³, bevorzugt 1,01 g/cm³ bis 1,30 g/cm³ eingestellt. Auch der Bereich zwischen 1,001 und 1,01 g/cm³ ist vorgesehen, z.B. 1,001 g/cm³; 1,002 g/cm³; 1,003 g/cm³; 1,004 g/cm³; 1,005 g/cm³; 1,006 g/cm³; 1,007 g/cm³; 1,008 g/cm³; 1,009 g/cm³. Die Osmolarität sollte darüber hinaus in einem Bereich von 280-330 mosmol/L, bevorzugt bei 300 mosmol/L liegen. In Betracht kommen mit Wasser kompatible Flüssigkeiten, deren Dichte über der Dichte des Wassers liegt. Die Dichte wird vorzugsweise bei 20°C gemessen, d.h. die Temperatur des Färbemittels soll 20!C betragen, wobei bei dieser Temperatur die Dichte gemessen wird. Die Dichte wird vorzugsweise mit einem Pyknometer gemessen. Ein bevorzugtes Pyknometer kann z.B. ein Mettler Toledo DA-100M Density Meter (RBE93409) sein (Bereich: 0-3 g/cm³, Genauigkeit: 0,001 g/cm³).

Mittel zur Einstellung der Dichte sind mit Wasser kompatible Flüssigkeiten, deren Dichte über der Dichte des Wassers liegt. Ein vorteilhaftes Mittel zur Erhöhung der Dichte ist schweres Wasser, D₂O, mit dem der Dichtewert auf den gewünschten Bereich eingestellt werden kann. Schweres Wasser zeichnet sich durch eine hervorragende Verträglichkeit aus. Es wird bis zu einer Konzentration von 20% in Wasser von Eukaryonten toleriert und führt nicht zu Reizungen im Anwendungsgebiet. Es ist mit Wasser in jeder beliebigen Konzentration mischbar, neigt nicht zum Absetzen oder zur Separation und weist bezüglich der Löslichkeit keine nachweisbaren Unterschiede gegenüber Wasser auf. Der Anteil an schwerem Wasser kann in der Zubereitung so eingestellt werden, dass der gewünschte Dichtewert von 1,01 g/cm³ bis 1,50 g/cm³, bevorzugt 1,01 g/cm³ bis 1,30 g/cm³, erreicht wird. Die geeignete Menge, die auch von den weiteren Inhaltsstoffen abhängt, kann durch einfache Versuche oder Berechnungen gefunden werden. Wenn schweres Wasser das die Dichte einstellende Mittel ist, wird es bevorzugt in einer Menge von 5-20%V/V, besonders bevorzugt in einer Menge von 13%V/V verwendet. Auch die Herstellung der Zubereitung mit schwerem Wasser ist sehr einfach und kann einfach durch Vermischen erfolgen aufgrund der guten Vermischbarkeit der beiden Bestandteile. Aus Wasser, schwerem Wasser und Farbstoff lässt sich daher einfach und schnell eine auf Dauer stabile und für den Zweck des selektiven Anfärbens der Membranen gut geeignete Zubereitung herstellen.

Ein weiteres Mittel, mit dem die Dichte eingestellt werden kann, ist ein Di- oder Polysaccharid. Polysaccharide eignen sich zur Erhöhung der Dichte und sind gut verfügbar. Außerdem sind sie toxikologisch unbedenklich und biologisch kompatibel. Unter Polysacchariden werden hier Moleküle verstanden, die aus mehr als zwei, bevorzugt mehr als 5, besonders bevorzugt mehr als 10 Saccharideinheiten aufgebaut sind. Obwohl allgemein Mono- und Disaccharide die Dichte erhöhen können, werden erfindungsgemäß bevorzugt nicht-reduzierende Disaccharide zur Erhöhung der Dichte eingesetzt. Die Verwendung von Monosacchariden und reduzierenden Disacchariden kann zu unerwünschten Wirkungen führen, so können diese z.B. in der zur Erhöhung der Dichte notwendigen Menge zytotoxisch sein. Nichtsdestotrotz ist auch Glukose als Mittel zur Einstellung der Dichte im Sinne der Erfindung vorgesehen, dann aber bevorzugt in einer Konzentration von bis zu (und inklusive) 5% (v/v), mehr bevorzugt in einer Konzentration von 4,9; 4,8, 4,7; 4,6; 4,5; 4,4; 4,3; 4,2; 4,1 oder 4,0% (v/v). Erfindungsgemäß geeignete nicht-reduzierende Disaccharide sind Saccharose oder Trehalose. Als geeignete Polysaccharide können lösliche Stärkederivate, wie Hydroxyethylstärke und Dextran genannt werden. Auch Mannitol ist vorgesehen. Als Polysaccharide sind solche Verbindungen geeignet, die neutral sind, nicht reduzierend wirken und sich in wässriger Lösung nicht abbauen.

Weitere Mittel zur Einstellung der Dichte sind neutrale Polymere wie Polyether, beispielsweise Polyethylenglykol (PEG), Polyvinylalkohol, Polyester, Polyacrylsäure Copolymer, Polyvinylpyrrolidon. Ebenso ist Hyaluronsäure oder Hyaluronsäurederivate wie Ester davon, zur Einstellung der Dichte vorgesehen. Auch Kombinationen der genannten Mittel sind gut geeignet, um die Dichte der erfindungsgemäßen Zubereitung einzustellen, z.B. eine Kombination aus schwerem Wasser und einem oder mehreren Polysacchariden. Die Menge an schwerem Wasser und/oder weiteren oder anderen die Dichte einstellenden Mitteln wird dabei so gewählt, dass die Dichte der fertig hergestellten Zubereitung im erforderlichen Bereich von 1,01 g/cm³ bis 1,50 g/cm³, bevorzugt 1,01 g/cm³ bis 1,30 g/cm³ liegt. Die Dichte der Zubereitung kann dabei nach jeder gängigen Methode bestimmt werden, wie sie dem Fachmann allgemein bekannt ist.

In einer weiteren bevorzugten Ausführungsform der vorliegenden Erfindung wird die Dichte des Färbemittels in einem Bereich von 0,50 g/cm³ bis 0,99 g/cm³, bevorzugt 0,80 g/cm³ bis 0,99 g/cm³ eingestellt. Die Osmolarität sollte darüber hinaus in einem Bereich von 280-330 mosmol/L, bevorzugt bei 300 mosmol/L liegen. In Betracht kommen mit Wasser kompatible Flüssigkeiten, deren Dichte unter der Dichte des Wassers liegt. Ein vorteilhaftes Mittel zur Erniedrigung der Dichte ist z. B. Alkohol in physiologisch verträglichen Mengen, mit dem der Dichtewert auf den gewünschten Bereich eingestellt werden kann. Auch der Bereich zwischen 1,001 und 1,01 g/cm³ ist als Dichte für das Färbemittel vorgesehen, z.B. 1,001 g/cm³; 1,002 g/cm³; 1,003 g/cm³; 1,004 g/cm³; 1,005 g/cm³; 1,006 g/cm³; 1,007 g/cm³; 1,008 g/cm³; 1,009 g/cm³.

Ferner ist vorgesehen, dass das Färbemittel in einer bevorzugten Ausführungsform der Erfindung mit einem viskoelastischen Stoff gemischt wird bzw. ein viskoelastischer Stoff dem Färbemittel zugesetzt wird. Die Viskosität eines solchen Färbemittels beträgt vorzugsweise mindestens 1,5 oder 2,0 mPa•s, mehr bevorzugt 2,2; 2,3; 2,4 oder 2,5 mPa•s. Vorzugsweise beträgt die Viskosität weniger als 18 mPa•s, mehr bevorzugt beträgt sie weniger als 9, 8, 7, 6, oder 5 mPa•s. Die Viskosität wird vorzugsweise bei einer Temperatur von 20°C gemessen, d.h. das Färbemittel soll eine Temperatur von 20°C bei der Messung haben.

Augenheilkundliche Operationen an und/oder mit und/oder unter Beteiligung der Hornhaut sind z.B. die hierin beschriebenen perforierenden und lamellierenden Verfahren der Keratoplastik. Dabei wird die Hornhaut oder Teile davon mit dem erfindungsgemäßen Färbemittel angefärbt und dann daran die entsprechende Operation durchgeführt wie hierin beschrieben.

Beispielsweise verhält es sich beim Färben der Hornhaut so, dass der ausführende Operateur die Hornhaut oder Teile davon des Auges des Transplantatempfängers (Transplantat-Akzeptor) und/oder Transplantatgebers (Transplantat-Donor) markieren kann, um dann entsprechende Stellen auszuschneiden, z.B. mechanisch oder mittels Excimer-Laser-Trepanation und mit einem Transplantat zu ersetzen. Es können bei der Hornhauttransplantation alle Schichten der Hornhaut transplantiert werden (perforierende Keratoplastik) oder nur spezielle Schichten der Hornhaut (lamellierende Keratoplastik). Um nun die Hornhaut bzw. Bereiche oder Stellen sowohl der Spender-Hornhaut als auch der Empfänger-Hornhaut davon sichtbar zu machen, kann das erfindungsgemäße Färbemittel verwendet werden, um z.B. die Hornhaut von außen zu markieren oder beim Vernähen der Spenderhornhaut mit der Empfängerhornhaut als Nahtmarker zu dienen.

Augenheilkundliche oder chirurgische Verfahren (Verfahren der Augenchirurgie), wie hierin beschrieben, umfassen insbesondere die Keratoplastik, umfassend Thermokeratoplastik, perforierende Keratoplastik und lamellierende Keratoplastik. Diese wird in der modernen Ophthalmologie bei einer Vielzahl spezifischer, invalidisierender Hornhauterkrankungen eingesetzt. Die meisten Hornhautübertragungen werden aus optischen Gründen durchgeführt, d.h. um Trübungen der normalerweise optisch klaren Hornhaut zu beseitigen. Eine Keratoplastik kann darüber hinaus erforderlich sein, um aktive Infektionsherde des Hornhautgewebes auszuräumen oder Defekte zu decken, wie sie beispielsweise nach schweren Entzündungen, Verletzungen oder Operationen vorkommen. In den meisten Fällen ist die Integrität der übrigen Strukturen des Auges erhalten und eine gute visuelle Rehabilitation möglich.

Bei den lamellierenden Techniken wird grundsätzlich zwischen den anterioren und den posterioren lamellierenden Keratoplastiken unterschieden, welche indikationsspezifisch eingesetzt werden können.

Bei der anterioren lamellierenden Keratoplastik (DALK = Deep anterior lamellar keratoplasty) wird die Trepanation der Empfängerhornhaut nicht vollständig, sondern nur bis in die tiefen Schichten des Stromas vorgenommen. Anschließend wird die Präparation möglichst bis auf die Descemet-Membran durchgeführt, so dass das Auge nicht eröffnet wird und die für die Prognose entscheidende Zellschicht, das Endothel des Empfängers bestehen bleibt. Das Einfügen des Spenderhornhautscheibchens erfolgt nach Entfernung des Endothels und der Descemet-Membran. Dieses Verfahren ist folglich nur geeignet, wenn das Endothel des Empfängers intakt ist. Eine klassische Indikation stellt der Keratokonus dar. Die tiefe Präparation der Hornhautlamelle beim Empfänger kann auch Pachymetrie-gesteuert mit dem Excimer-Laser vorgenommen werden (PALK = Pachymetrie-assistierte Laser-Keratoplastik).

Die posteriore lamellierende Keratoplastik ist indiziert, wenn Epithel und Stroma des Patienten intakt sind, jedoch eine endotheliale Erkrankung vorliegt. Klassische Indikationen sind die Fuchs'sche Endotheldystrophie und die bullöse Keratopathie als Folge einer Dekompensation des Endothels nach Katarakt-Operation. Es gibt zwei grundsätzliche Techniken, die DSAEK (Descemet's stripping with automated endothelial keratoplasty) und die DMEK (Descemet's membrane endothelial keratoplasty).

Bei der DSAEK-Technik wird in einer künstlichen Vorderkammer mit Hilfe eines Mikrokeratoms aus der Spenderhornhaut eine dünne hintere Stromalamelle mit intaktem Endothel gewonnen. Dieses Präparat wird in die Vorderkammer des Empfängers gebracht und dort von innen an die Hornhaut gelegt, nachdem dort zuvor das Endothel entfernt wurde.

Die DMEK-Methode wird angewendet, um ausschließlich das Endothel mit der Descemet-Membran zu transplantieren. Dabei wird von der Spenderhornhaut nur die Descemet-Membran mit dem Endothel abpräpariert, um dann in der Vorderkammer des Empfängers, bei dem zuvor das Hornhautendothel entfernt wurde, eingebracht und angelegt zu werden. Dies erfordert viel Geduld und Geschick beim Operateur.

Insgesamt könnten grundsätzliche Vorteile der lamellierenden Verfahren gegenüber der perforierenden Keratoplastik erwartet werden: geringeres intraoperatives Risiko, schnellere visuelle Rehabilitation, weniger irreguläre Astigmatismus-Entwicklung, weniger Abstoßungsreaktionen, eine geringere Transplantatversager-Quote, längere Überlebenszeit der Transplantate und eine geringere Re-Keratoplastik-Rate. Allerdings sind die lamellierenden Techniken mit einer längeren Lernkurve und größerem Zeit- und Geräteaufwand verbunden, außerdem sind sie weniger standardisiert als die perforierende Keratoplastik.

Es wird gemäß einem weiteren Aspekt der vorliegenden Erfindung die Verwendung des Färbemittels als chirurgisches Hilfsmittel für augenheilkundliche Operationen an oder unter Beteiligung der Hornhaut zur Verfügung gestellt.

Des Weiteren beschreibt die vorliegende Erfindung die Verwendung eines hierin beschriebenen Farbstoffs, insbesondere eines Triphenylmethanfarbstoffs zur Herstellung eines erfindungsgemäßen biokompatiblen Färbemittels zum Anfärben der Hornhaut und/oder als Hilfsmittel zum Anfärben der Hornhaut bei augenheilkundlichen Operationen wie hierin beschrieben an und/oder mit der Hornhaut.

In einer bevorzugten Ausführungsform umfasst ein erfindungsgemäßes Färbemittel zwei oder mehr Farbstoffe ausgewählt aus der Gruppe bestehend aus Triphenylmethanfarbstoffen, Azofarbstoffen, Naturfarbstoffen, Cyaninfarbstoffen, und/oder Naphtocyaninfarbstoffen, wie z.B. in der WO 2011/151287 beschrieben.

Das erfindungsgemäße Färbemittel ist vorzugsweise ein Medizinprodukt, das vorzugsweise einen pharmazeutisch verträglichen Träger umfasst. Das Medizinprodukt liegt vorzugsweise in wässriger Form vor. Alternativ dazu kann es auch lyophilisiert vorliegen und wird bei Bedarf rekonstituiert.

Der hierin verwendete Begriff "pharmazeutisch verträglich" bzw. "pharmazeutisch annehmbar" beinhaltet, dass die entsprechende Substanz physiologisch kompatibel sowie biokompatibel ist und im erfindungsgemäßen Färbemittel bei den hierin beschriebenen ophthalmologischen Eingriffen ohne schädigenden Einfluss auf den Patienten bleibt.

Gegenüber dem herkömmlichen Trypanblau, welches teratogene oder mutagene Wirkung hat (Cahen RL: Evaluation of the teratogenicity of drugs, Clin Pharmacol. Ther, 1964, 5,480-514 und Produktinformation BLURHEXTM, Dr. Agarwal's Pharma Ltd. Chennai (Indien)), ICG, welches fototoxisch (Phototoxicity of Indocyanine Green on Human Retinal Pigment Epithelium in Vitro and its Reduction by Lutein. Wu W.-C. et al., Photochemistry and Photobiology 2005; 81: 537-540) und zytotoxisch (Comparative studies on the retinal toxicity of trypan blue and indocyanin green. Ito T. et al., Invest Ophthalmol Vis Sci 2004; 45: 3669 - B130) ist, sowie Gentianaviolett, welches Hornhautödeme verursacht (Gentian violet solution for staining the anterior capsule. Ünlü K. et al.; J Cataract Refract Surg. 2000; 26: 1228-1232 und Experimental staining of the anterior lens capsule in albino rabbits, Gamal Eldin et. al.; J Cataract Refract Surg. 1999; 25: 1289-1294), besitzt die erfindungsgemäß biokompatible Lösung keine Zytotoxizität.

Zum Nachweis fehlender Zytotoxizität wurden nach dem vorgeschriebenen Zytotoxizitätstest (Normenreihe ISO 10993) Mauszellen, vorzugsweise L929 Zellen (ATCC No. CCL1, NCTC clone 929) mit dem erfindungsgemäßen Färbemittel mit unterschiedlichen Konzentrationen an Farbstoff, (z.B. ausgehend von einer 0,6 mg/ml Lösung werden Verdünnungen angefertigt, wie z.B. 13,2%, 19,8%, 29,6%, 44,4%, 66,7%, und die ursprüngliche Lösung mit 100%) über ca. 68 bis 72 Stunden im Brutschrank bei ca. 37°C und 5 % CO₂ in Kontakt gebracht. Vorzugsweise werden die Zellen in Kulturgefäßen, insbesondere Zellkulturflaschen mit einer Fläche von vorzugsweise 75 cm² oder 175 cm² gezüchtet und in diesen Kulturgefäßen auch mit dem Färbemittel in Kontakt gebracht/kultiviert. Vorzugsweise enthält das Kulturmedium ein dem Fachmann bekanntes Kulturmedium wie z.B. DMEM (z.B. von Invitrogen), auch 10% FKS (Fötales Kälberserum), z.B. 10% FCS-Gold von PAA. Die Wachstumshemmung wird mit an sich bekannten Verfahren bestimmt. Die Vitalität der Zellen und eine abgeleitete Zytotoxizität werden durch Bestimmung des Proteingehalts der behandelten Zellkulturen gegenüber unbehandelten Kontrollkulturen quantitativ bestimmt. Mit einem Standard-Verfahren, vorzugsweise BCA-Färbung wird der Proteingehalt kolorimetrisch ermittelt. Dabei zeigt sich, dass eine Zytotoxizität in signifikanter Höhe entsprechend einer Wachstumshemmung von mehr als 30% im Vergleich zu L929-Zellen, die nicht mit dem Färbemittel in Kontakt gebracht/kultiviert wurden, nicht vorhanden ist. Als Positivkontrolle kann z.B. Medium (z.B. DMEM) mit FCS, z.B. 10% und 5% DMSO mit den entsprechenden Testzellen verwendet werden, um zu sehen, ob die Testzellen im Wachstum inhibiert werden. Als Negativkontrolle werden Testzellen mit Medium, z.B. DMEM mit 10% FCS verwendet, also keine Farbstofflösung hinzugegeben.

Zusätzlich oder alternativ kann die fehlende Zytotoxizität des Färbemittels anhand von ARPE 19 Zellen mit unterschiedlichen Konzentrationen an Farbstoff (z.B. 0,5 g/L, 0,25 g/L und 0,1 g/L) evaluiert werden. Die Expositionszeiten auf den Zellkulturen betragen vorzugsweise 30, 60, 120 und 300 Sekunden. Es werden vorzugsweise Doppelbestimmungen durchgeführt. Die Evaluation erfolgt vorzugsweise mittels eines MTT-Assays. Die Zellreihen werden hierzu vorzugsweise in 12-well-Platten zu 100% Konfluenz gebracht. Es wird pro Well je 1 mL gelöster Farbstoff in der entsprechenden Zeit aufgebracht. Nach der Farbstoffapplikation wird den Zellen 24 Stunden Zeit für eine entsprechende Reaktion gegeben. Dann wird das MTT-Assay durchgeführt und im ELISA-Reader ausgewertet. Pro Zelllinie und Lösungsmittel werden vorzugsweise 3 voneinander unabhängige Versuchsreihen durchgeführt.

Vorzugsweise verursacht das erfindungsgemäße Färbemittel kein Hornhautödem, wie z.B. Gentianviolett (Enzianblau), siehe Ünlü et al. oder Gamal Eldin et al., beide oben zitiert.

Der Begriff "fehlende Zytotoxizität", "Biokompatibilität" oder "biokompatibel" beinhaltet, dass das erfindungsgemäße Färbemittel in dem vorbeschriebenen Zytotoxizitätstest (z.B. gemäß Normenreihe ISO 19993) unter Verwendung der angegebenen Mengen/Konzentrationen bei L929-Zellen keine Wachstumshemmung von mehr als 30% unter den beschriebenen Bedingungen im Vergleich zu unbehandelten L-929-Zellen hervorruft bzw. unter Verwendung der angegebenen Mengen/Konzentrationen bei ARPE-19-Zellen keine Wachstumshemmung von mehr als 20% unter den beschriebenen Bedingungen im Vergleich zu unbehandelten ARPE-19-Zellen hervorruft. Durch die hohe Biokompatibilität des Färbemittels besteht ein berechtigter Grund zur Annahme, dass bisher bekannte Nebenwirkungen wie z.B. Ödeme verhindert werden können.

Das erfindungsgemäße Färbemittel wird also vorzugsweise in einer solchen Konzentration verwendet, die in den hierin beschriebenen Tests keine Zytotoxizität aufweist und aber noch eine ausreichende Färbung der Hornhaut bewirken kann. Der Fachmann ist in der Lage, die Färbung an Hand bekannter Testverfahren zu bestimmen.

Es wird ein biokompatibles Färbemittel wie in den Ansprüchen definiert zur Verfügung gestellt, welches als Hauptkomponente mindestens einen Farbstoff enthält, der ausgewählt ist aus Triphenylmethanfarbstoffen und/oder Azofarbstoffen und/oder Cyaninfarbstoffen und/oder Naturfarbstoffen, oder ein pharmazeutisch verträgliches Salz und/oder Hydrat dieser Farbstoffe, zum Färben der Hornhaut, bzw. deren Bestandteile, sowie einen pharmazeutisch akzeptablen Träger.

Die Begriffe "Einfärben", "Anfärben", oder "Färben", wenn sie hierin verwendet werden, können gleichbedeutend verwendet werden. Jeder der Begriffe beinhaltet, dass eine ophthalmische Membran, insbesondere die Hornhaut entweder selbst durch das erfindungsgemäße Färbemittel gefärbt/angefärbt wird (sog. Positivfärbung) und/oder dass zwar die ophthalmische Membran, insbesondere die Hornhaut selbst nicht gefärbt wird, aber dafür ophthalmische Membranen, die um die nicht selbst gefärbte ophthalmische Membran, insbesondere die Hornhaut gelegen sind und/oder an die selbst nicht gefärbte ophthalmische Membran, insbesondere die Hornhaut angrenzen (sog. negative Färbung). Das Anfärben kann dabei die ganze oder Teile der ophthalmischen Membran, insbesondere der Hornhaut betreffen, z.B. das Anzeichnen bestimmter Bereiche der Hornhaut oder das sogenannte Hornhaut-Tattooing (Hornhaut-Tätowierung).

Entsprechend einer Ausführungsform der vorliegenden Erfindung, wird das Färbemittel zum Färben der Hornhaut, bzw. deren Bestandteile, zum Zeitpunkt der Entfernung und/oder vor dem Zeitpunkt der Entfernung eines Teils der Hornhaut, bzw. eines Teils deren Bestandteile, zur Verfügung gestellt.

Das Färbemittel kann zum Anfärben des Hornhautstromas, insbesondere für die periphere stromale Schicht der Spenderhornhaut bei einer DLEK-Operation verwendet werden. Dadurch wird die Kontaktfläche zwischen Spender- und Empfängerhornhaut für den Operateur sichtbar gemacht, was die Operation positiv unterstützt. Darüber hinaus wird sichergestellt, dass die Schichten der Spenderhornhaut in der richtigen Orientierung in die Empfängerhornhaut eingebaut werden, um somit das Gelingen der Operation zu gewährleisten.

Das Färbemittel kann zum Anfärben der Descemet-Membran der Hornhaut, insbesondere bei einer perforierenden Keratoplastik verwendet werden. Durch die Färbung der Spender- und/oder Empfängermembran kann die Anpassung der Schnittkanten der Spender-und Empfängermembran unterstützt werden, was eine Verbesserung der Transplantatstabilität sowie eine Verminderung eines durch die Operation verursachten Astigmatismus zur Folge hat. Zusätzlich können bei der perforierenden Keratoplastik nach Entfernung der Empfängerhornhaut Reste der Descemet-Membran durch das Färbemittel sichtbar gemacht und aus dem Auge entfernt werden, um Komplikationen nach dem Eingriff zu minimieren.

Das Färbemittel kann zum Anfärben des Endotheliums der Hornhaut, insbesondere bei der Transplantation der Descemet'schen Membran verwendet werden. Dadurch wird wiederum sichergestellt, dass die Schichten der Spenderhornhaut in der richtigen Orientierung in die Empfängerhornhaut eingebaut werden, um somit das Gelingen der Operation zu gewährleisten.

Die Verwendung des erfindungsgemäßen Färbemittels kann den Einsatz von Halogen- und/oder Xenonlicht während des chirurgischen Eingriffs positiv beeinflussen. Insbesondere ist der im Färbemittel verwendete Farbstoff im Gegensatz zu ICG nicht fototoxisch.

Weiterhin stell die Erfindung ein Verfahren zum ex vivo Färben der menschlichen oder tierischen Hornhaut oder Teilen davon bzw. deren Bestandteile, umfassend Färben der entnommenen Hornhaut oder Teilen davon bzw. deren Bestandteile mit einem Färbemittel, enthaltend mindestens einen Farbstoff, der ausgewählt ist aus Triphenylmethanfarbstoffen, Azofarbstoffen, Cyaninfarbstoffen, Naphtocyaninfarbstoffen und/oder Naturfarbstoffen. Vorzugsweise färbt das Färbemittel das Stroma der Hornhaut oder Teilen davon.

Ferner ist es möglich, ein viskoelastisches Material, beispielsweise Hyaluronsäure, welches als Hilfsmittel bei der ophthalmologischen Chirurgie zum Einsatz kommt, mit der wässrigen Färbemittellösung einzufärben. Vorzugsweise wird ein Triphenylmethanfarbstoff, z.B. Acid Violet 17 oder BBG, oder ein Azofarbstoff mit einem viskoelastischen Material, wie z.B. Hyaluronsäure oder Derivaten davon, wie z.B. Ester, gemischt. Vorzugsweise wird mittels der Hyaluronsäure die Dichte wir hierin so eingestellt, dass die Lösung schwerer ist als Wasser, z.B. schwerer als 1,001 g/cm³, vorzugsweise nicht schwerer als 1,01 g/cm3, z.B. 1,002; 1,003; 1,004; 1,005; 1,006; 1,007; 1,008; 1,009 g/cm³.

Darüber hinaus ist es auch möglich mit dem erfindungsgemäßen Färbemittel eine andere, in der ophthalmischen Chirurgie verwendete, physiologisch kompatible Flüssigkeit zu färben. Diese Flüssigkeiten können insbesondere semifluorierte Alkane und/oder perfluorierte Carbone sein bzw. diese beinhalten.

Weiterhin kann das erfindungsgemäße Färbemittel auch zum Anfärben von ophthalmischen Flüssigkeiten, wie z.B. Glaskörpertamponaden, Silikonöle oder semifluorierte Alkane und/oder perfluorierte Carbone (siehe EP 859751 oder WO 2011/151079) verwendet werden. Dementsprechend stellt die vorliegende Erfindung eine ophthalmische Flüssigkeit bereit, die mit dem erfindungsgemäßen Färbemittel versetzt ist bzw. ein Verfahren zum Herstellen einer gefärbten ophthalmischen Flüssigkeit, umfassend Versetzen der ophthalmischen Flüssigkeit mit dem erfindungsgemäßen Färbemittel und Erhalten der gefärbten ophthalmischen Flüssigkeit. Die ophthalmische Flüssigkeit kann z.B. eine bei einer Augenoperation zu verwendende viskoelastische Flüssigkeit sein.

Mit den erfindungsgemäßen Färbemitteln lassen sich neben den bereits beschriebenen, einfachen Färbungen auch Mehrfachfärbungen oder Negativfärbungen durchführen, um z.B. die Vollständigkeit das Entfernens einer Membran aus dem Auge sicherzustellen.

Gemäß der Farbzusammensetzung der vorliegenden Erfindung wird es ermöglicht, schwer zu erkennende ophthalmische Membranen klarer sichtbar zu machen und die Sicherheit während der Operation zu erhöhen.

Das Färbemittel kann in Form eines Kits mit einem Lösungsmittel und Wirkstoffpulver, oder als Lösung, die vorzugsweise in eine Spritze gefüllt wurde, vorgelegt werden. Am meisten wird bevorzugt, dass es in Form einer Lösung bereitet wird, aber es ist nicht darauf beschränkt. Das Kit wird vorzugsweise in einem Verfahren der Augenchirurgie wie hierin beschrieben verwendet. Das Färbemittel des Kits ist vorzugsweise in einem Container oder in einer Spritze gefüllt vorgelegt.

Bezüglich eines weiteren Aspekts der vorliegenden Erfindung, wird eine Zusammensetzung wie in den Ansprüchen definiert zur Nutzung in einem Verfahren zum Färben und Entfernen und/oder Einsetzen der Hornhaut bzw. deren Bestandteile zur Verfügung gestellt, welches die Schritte des Herstellens eines Färbemittels, welches mindestens einen Farbstoff enthält, der ausgewählt ist aus Triphenylmethanfarbstoffen und/oder Azofarbstoffen und/oder Cyaninfarbstoffen und/oder Naturfarbstoffen, oder ein pharmazeutisch verträgliches Salz und/oder Hydrat dieser Farbstoffe, als Hauptkomponente aufweist; des Färbens der Hornhaut bzw. deren Bestandteile unter Benutzung einer vorher bestimmten Konzentration dieses Färbemittels; und des Entfernens und/oder Einsetzens zumindest eines Teils der gefärbten Hornhaut bzw. deren Bestandteile umfasst.

Für eine Ausführungsform der vorliegenden Erfindung kann zum Färben der Hornhaut bzw. deren Bestandteile jede Methode verwendet werden, die dem Fachmann des Standes der Technik leicht verständlich ist, zum Beispiel, Auftragung, Injektion, Infusion, und/oder Spülung.

Bezüglich eines weiteren Hauptaspekts der vorliegenden Erfindung, wird eine Verwendung des erfindungsgemäßen Färbemittels zur Behandlung ophthalmischer Erkrankungen, insbesondere der Hornhaut, wie hierin beschrieben, zur Verfügung gestellt.

Außerdem wird gemäß eines weiteren Hauptaspekts der vorliegenden Erfindung, die Verwendung des erfindungsgemäßen Färbemittels als chirurgisches Hilfsmittel für augenheilkundliche Operationen, insbesondere augenheilkundliche Operationen an der Hornhaut wie hierin beschrieben zur Verfügung gestellt.

Weiterhin wird das Färbemittel, und/oder dessen Verwendung in einem Färbeverfahren der vorliegenden Erfindung als Teil einer augenheilkundlichen Operation genutzt werden.

Dementsprechend betrifft die vorliegende Erfindung auch ein Verfahren zum Färben der Hornhaut oder Teilen davon bzw. deren Bestandteile, umfassend Zubereiten eines erfindungsgemäßen Färbemittels und Färben der Hornhaut bzw. deren Bestandteile bei der Hornhauttransplantation, z.B. perforierende Keratoplastik, vordere und hintere lamellierende Keratoplastik, der Corneamarkierung, etc..

Das erfindungsgemäße Färbemittel ist vorzugsweise ein Medizinprodukt, das vorzugsweise einen pharmazeutisch verträglichen Träger umfasst. Das Medizinprodukt liegt vorzugsweise in wässriger Form vor. Alternativ dazu kann es auch lyophilisiert vorliegen und wird bei Bedarf rekonstituiert.

Gegebenenfalls umfasst das Verfahren auch das Zubereiten eines erfindungsgemäßen Färbemittels, ehe das Färben wie hierin beschrieben durchgeführt wird.

Des Weiteren betrifft die vorliegende Erfindung auch ein Verfahren zum Färben und Entfernen von aus dem Auge zu entfernenden Membranen, insbesondere der ophthalmischen Membranen bei Netzhaut- oder Glaskörperchirurgie, umfassend Färben von einer oder mehreren von aus dem Auge zu entfernenden Membran(en), insbesondere der ophthalmischen Membranen bei Netzhaut- oder Glaskörperchirurgie und Entfernen der zu entfernenden Membranen, insbesondere der ophthalmischen Membranen bei Netzhaut- oder Glaskörperchirurgie. Das Entfernen erfolgt vorzugsweise durch sogenanntes Peeling.

Entsprechend einer bevorzugten Ausführungsform der vorliegenden Erfindung handelt es sich bei den augenheilkundlichen Operationen um Operationen zur Behandlung von Makula-Loch, Netzhautablösung bedingt durch ein hochmyopes Auge, epiretinale Membran, proliferative diabetische Retinopathie, makuläres Ödem wie z.B. diabetisches makuläres Ödem, und proliferative Vitreo-Retinopathie, sowie spezifische Katarakte, wie hypermaturer Katarakt und kongenitaler Katarakt, darüber hinaus kann das erfindungsgemäße Färbemittel auch bei der perforierenden Keratoplastik, der vorderen und hinteren lamellierenden Keratoplastik und der Corneamarkierung, etc., benutzt werden.

Weiterhin werden die hierin beschriebenen augenheilkundlichen Operationen gemäß einer bevorzugten Ausführungsform der vorliegenden Erfindung an Augen von Säugetieren, wie z.B. Hunden, Katzen, Pferden und mehr bevorzugt an Augen von Menschen durchgeführt.

Weitere Aspekte der Erfindung sind:
1. Färbemittel zum Anfärben einer ophthalmischen Membran enthaltend Acid Violet 17, ein pharmazeutisch verträgliches Salz und/oder Hydrat davon und einen pharmazeutisch akzeptablen Träger.
2. Färbemittel nach Aspekt 1 wobei die ophthalmische Membran die innere Grenzmembran und/oder die vordere und/oder hintere Linsenkapsel und/oder die Hornhaut bzw. deren Bestandteile ist.
3. Färbemittel nach Aspekt 1 oder 2, wobei das Färbemittel Acid Violet 17 in einer Konzentration von 1 x 10⁻³ - 10 g/L, bevorzugt in einer Konzentration von 0.005 -1.5 g/L und am meisten bevorzugt in einer Konzentration von 0,10 - 0,50 g/L für die Färbung der ILM, einer Konzentration von 0,20 bis 1,0 g/L für die Färbung der Linsenkapsel, sowie einer Konzentration von 0,1 bis 1,5 g/L für die Färbung der Hornhaut bzw. deren Bestandteile beinhaltet.
4. Färbemittel nach einem der Aspekte 1 bis 3, wobei der pharmazeutisch akzeptable Träger eine physiologisch kompatible wässrige Lösung mit einem pH von 6,8 bis 7,8 ist.
5. Färbemittel nach einem der vorangegangenen Aspekte, wobei die Dichte des Färbemittels in einem Bereich von 1,01 g/cm³ bis 1,5 g/cm³, bevorzugt 1,01 g/cm³ bis 1,3 g/cm³ eingestellt wird.
6. Färbemittel nach Aspekt 5, wobei das die Dichte einstellende Mittel D₂O ist.
7. Färbemittel nach einem der vorangegangenen Aspekte, wobei die Dichte des Färbemittels in einem Bereich von 0,50 g/cm³ bis 0,99 g/cm³, bevorzugt 0,80 g/cm³ bis 0,99 g/cm³ eingestellt wird.
8. Färbemittel nach einem der vorangegangenen Aspekte, wobei das Färbemittel eine Osmolarität von 280-330 mosmol/L, bevorzugt bei 300 mosmol/L aufweist.
9. Färbemittel zur Verwendung in einem Verfahren der Augenchirurgie, umfassend das Einfärben der ophthalmischen Membran und die Durchführung der Entfernung der ophthalmischen Membran, wobei das Färbemittel Acid Violet 17, ein pharmazeutisch verträgliches Salz und/oder Hydrat davon und einen pharmazeutisch akzeptablen Träger umfasst.
10. Färbemittel nach Aspekt 9 wobei die ophthalmische Membran die innere Grenzmembran und/oder die vordere und/oder hintere Linsenkapsel und/oder die Hornhaut bzw. deren Bestandteile ist.
11. Färbemittel nach Aspekt 9 oder 10, wobei das Färbemittel Acid Violet 17 in einer Konzentration von 1 x 10⁻³ - 10 g/L, bevorzugt in einer Konzentration von 0,005 -1.5 g/L und am meisten bevorzugt in einer Konzentration von 0.10 - 0.50 g/L für die Färbung der ILM, einer Konzentration von 0,20 bis 1,0 g/L für die Färbung der Linsenkapsel, sowie einer Konzentration von 0,1 bis 1,5 g/L für die Färbung der Hornhaut bzw. deren Bestandteile beinhaltet.
12. Färbemittel nach einem der Aspekte 9 bis 11, wobei der pharmazeutisch akzeptable Träger eine physiologisch kompatible wässrige Lösung mit einem pH von 6,8 bis 7,8 ist.
13. Färbemittel nach einem der Aspekte 9 bis 12, wobei die Dichte des Färbemittels in einem Bereich von 1,01 g/cm³ bis 1,5 g/cm³, bevorzugt 1,01 g/cm³ bis 1,3 g/cm³ eingestellt wird.
14. Färbemittel nach Aspekt 13, wobei dass die Dichte einstellende Mittel D₂O ist.
15. Färbemittel nach einem der Aspekte 9 bis 12, wobei die Dichte des Färbemittels in einem Bereich von 0,50 g/cm³ bis 0,99 g/cm³, bevorzugt 0,80 g/cm³ bis 0,99 g/cm³ eingestellt wird.
16. Färbemittel nach einem der Aspekte 9 bis 15, wobei das Färbemittel eine Osmolarität von 280-330 mosmol/L, bevorzugt bei 300 mosmol/L aufweist.
17. Kit enthaltend ein Lösungsmittel und Acid Violet 17, ein pharmazeutisch verträgliches Salz und/oder Hydrat davon als Wirkstoffpulver, oder enthaltend ein Färbemittel wie in den Aspekten 1 bis 8 definiert, welches vorzugsweise in eine Spritze gefüllt vorgelegt wird.
18. Kit nach Aspekt 17 zur Verwendung in einem Verfahren der Augenchirurgie nach einem der Aspekte 9 bis 16.
19. Medizinprodukt, umfassend das wie in den Aspekten 1 bis 16 definierte Färbemittel.
20. Biokompatibles Färbemittel zum Anfärben der Hornhaut oder Teilen davon, bzw. deren Bestandteile enthaltend mindestens einen Farbstoff, der ausgewählt ist aus Triphenylmethanfarbstoffen, und/oder Azofarbstoffen, und/oder Cyaninfarbstoffen, und/oder Naphtocyaninfarbstoffen, und/oder Naturfarbstoffen, oder ein pharmazeutisch verträgliches Salz und/oder Hydrat dieser Farbstoffe, sowie einen pharmazeutisch akzeptablen Träger.
21. Färbemittel nach Aspekt 20, wobei der Farbstoff ein Triphenylmethanfarbstoff oder ein pharmazeutisch verträgliches Salz und/oder Hydrat davon ist.
22. Färbemittel nach Aspekt 21, wobei der Farbstoff BBG oder Acid Violet 17 oder ein pharmazeutisch verträgliches Salz und/oder Hydrat davon ist.
23. Färbemittel nach einem der Aspekte 20 bis 22, wobei der Farbstoff in einer Konzentration von 1 x 10⁻³ - 10 g/L, bevorzugt in einer Konzentration von 0,1 bis 1,5 g/L vorliegt.
24. Färbemittel nach einem der vorhergehenden Aspekte, wobei der pharmazeutisch akzeptable Träger eine physiologisch kompatible wässrige Lösung mit einem pH von 6,8 bis 7,8 ist.
25. Färbemittel nach einem der vorhergehenden Aspekte, wobei das Färbemittel eine Osmolarität von 280-330 mosmol/L, bevorzugt bei 300 mosmol/L aufweist.

## Patentansprüche

1. Biokompatibles Färbemittel zur Verwendung in einem Verfahren der Augenchirurgie, insbesondere der Hornhautchirurgie, umfassend das Einfärben der Hornhaut oder Teilen davon bzw. deren Bestandteile und die Durchführung der Entfernung und/oder des Einsetzens zumindest eines Teils der gefärbten Hornhaut bzw. deren Bestandteile oder der gesamten Hornhaut, wobei das Färbemittel mindestens einen Farbstoff, der ausgewählt ist aus Triphenylmethanfarbstoffen, und/oder Azofarbstoffen und/oder Cyaninfarbstoffen und/oder Naphtocyaninfarbstoffen und/oder Naturfarbstoffen, oder ein pharmazeutisch verträgliches Salz und/oder Hydrat dieser Farbstoffe, und gegebenenfalls einen pharmazeutisch akzeptablen Träger umfasst.

2. Färbemittel zur Verwendung nach Anspruch 1, wobei der Farbstoff ein Triphenylmethanfarbstoff oder ein pharmazeutisch verträgliches Salz und/oder Hydrat davon ist.

3. Färbemittel zur Verwendung nach Anspruch 2, wobei der Farbstoff BBG oder Acid Violet 17 oder ein pharmazeutisch verträgliches Salz und/oder Hydrat davon ist.

4. Färbemittel zur Verwendung nach einem der vorangegangenen Ansprüche, wobei der Farbstoff in einer Konzentration von 1 x 10⁻³ - 10 g/L, bevorzugt in einer Konzentration von 0,1 bis 1,5 g/L vorliegt.

5. Färbemittel zur Verwendung nach einem der vorangegangenen Ansprüche, wobei der pharmazeutisch akzeptable Träger eine physiologisch kompatible wässrige Lösung mit einem pH von 6,8 bis 7,8 ist.

6. Färbemittel zur Verwendung nach einem der vorangegangenen Ansprüche, wobei das Färbemittel eine Osmolarität von 280-330 mosmol/L, bevorzugt bei 300 mosmol/L aufweist.

7. Färbemittel zur Verwendung nach einem der vorangegangenen Ansprüche, wobei die Dichte des Färbemittels in einem Bereich von 1,001 g/cm³ bis 1,5 g/cm³, bevorzugt 1,01 g/cm³ bis 1,3 g/cm³ eingestellt wird.

8. Färbemittel zur Verwendung nach Anspruch 7, wobei das die Dichte einstellende Mittel D₂O Hyaluronsäure oder Polyethylenglykol ist.

9. Kit enthaltend ein Lösungsmittel und einen Farbstoff zur Herstellung eines Färbemittels wie in den Ansprüchen 1 bis 8 definiert als Wirkstoffpulver, oder enthaltend ein Färbemittel wie in den Ansprüchen 1 bis 8 definiert, welches vorzugsweise in einer Spritze gefüllt vorgelegt wird.

10. Kit nach Anspruch 9, wobei das Färbemittel in einem Container oder in einer Spritze gefüllt vorgelegt wird.

11. Kit nach Anspruch 9 oder 10 zur Verwendung in einem Verfahren der Augenchirurgie nach einem der Ansprüche 1 bis 8.

12. Medizinprodukt, umfassend das wie in den Ansprüchen 1 bis 8 definierte Färbemittel.

13. Verfahren zum ex vivo Färben der menschlichen oder tierischen Hornhaut oder Teilen davon bzw. deren Bestandteile, umfassend Färben der entnommenen Hornhaut oder Teilen davon bzw. deren Bestandteile mit einem Färbemittel, enthaltend mindestens einen Farbstoff, der ausgewählt ist aus Triphenylmethanfarbstoffen, und/oder Azofarbstoffen und/oder Cyaninfarbstoffen und/oder Naphtocyaninfarbstoffen und/oder Naturfarbstoffen.

14. Verfahren nach Anspruch 13, wobei das Färbemittel das Stroma der Hornhaut oder Teilen davon färbt.
